# DEMANDE DE BREVET EUROPEEN

(11) **EP 2 767 246 A1**
(43) Date de publication de la demande: **20.08.2014**
(21) Numéro de dépôt: 14161014.7
(22) Date de dépôt: 17.02.2010
(51) Int. Cl.: A61B 17/80, A61B 17/00, A61B 19/00

(54) **PATIENTENSPEZIFISCHE ANORDNUNG MIT MINDESTENS ZWEI IHRERSEITS PATIENTENSPEZIFISCH AUSGELEGTEN OSTEOSYNTHESEPLATTEN**

(30) Priorité: 17.02.2009 FR 0900722
(62) Demande divisionnaire de: 10708241.4
(71) Demandeur: OBL, 92320 Chatillon (FR)
(72) Inventeur: Longepied, Patrice, F-92330 Sceaux (FR)
(74) Mandataire: EIP

(57) **Abrégé**

La présente invention est relative à un ensemble sur mesure (1) d'au moins deux plaques d'ostéosynthèse (2), par exemple de types droites en I et/ou en L et/ou en croix et/ou en forme de patte-d'oie, elles-mêmes préparées sur mesure en fonction de l'opération à réaliser et de la future anatomie du patient. Chaque plaque d'ostéosynthèse (2) a une courbure préconfigurée en relation avec une position que la plaque occupera lorsqu'elle sera fixée aux première et deuxième parties d'os de la mâchoire, la courbure étant ajustée sur la base d'une future anatomie souhaitée de la mâchoire, et en ce que l'ensemble comprend une partie de raccordement (3) pour raccorder conjointement lesdites plaques d'ostéosynthèse.

## Description

La présente invention est relative à un ensemble sur mesure d'au moins deux plaques d'ostéosynthèse, par exemple de types droites en I et/ou en L et/ou en croix et/ou en forme de patte-d'oie, elles-mêmes préparées sur mesure en fonction, d'une part, de l'opération à réaliser sur une mâchoire ou sur les deux mâchoires d'un patient et, d'autre part, de la future anatomie dudit patient.

Elle a également trait à un procédé de mise en place dudit ensemble sur mesure sur le patient.

On sait que, dans le cadre de la chirurgie plastique, en particulier orthognatique et traumatique, ou encore lors de certaines interventions chirurgicales, et notamment lors d'une intervention d'ostéosynthèse maxillomandibulaire - que cette dernière ne modifie pas l'occlusion ou qu'elle ne modifie pas les rapports occlusiaux ou encore qu'elle soit totale, en modifiant une arcade alvéolaire, ou segmentaire en n'intéressant qu'une partie de l'arcade - il est toujours nécessaire de recourir à la pose de plaques d'ostéosynthèse, c'est-à-dire de plaques de fixation vissées entre les parties osseuses qui se trouvent alors disjointes.

De telles plaques sont bien connues, en particulier du brevet français publié sous le n° 2.531.855 ou encore du brevet français publié sous le n° 2.725.124.

Ces plaques d'ostéosynthèse, qui sont le plus souvent réalisées en titane, sont tordues à la main par le chirurgien, avec des pinces, afin qu'elles s'adaptent parfaitement à la future anatomie du patient.

On sait par ailleurs réaliser, à partir des données d'un scanner ou d'une IRM, la simulation de l'ostéotomie grâce à laquelle on peut, au choix, avancer ou reculer la pièce osseuse du maxillaire et/ou de la mandibule jusqu'à, comme cela est souhaité, obtenir la disposition des dents au patient en regard les unes des autres.

Avant de procéder à une telle opération d'occlusion dentaire, on sait simuler par avance les résultats de l'opération sur le patient et l'on peut en conséquence préparer sur mesure des plaques d'ostéosynthèse de formes diverses qui devront être vissées aux endroits appropriés dans les éléments osseux d'une ou des deux mâchoires du patient, jusqu'à cicatrisation de l'os.

Cette préparation va jusqu'à donner elle aussi, ainsi qu'il a été dit en préliminaire, toutes les courbures voulues aux plaques d'ostéosynthèse, et ce afin d'adapter parfaitement ces dernières à la future anatomie du patient.

Cela étant, et ce problème n'a pas encore été résolu, rien ne permet d'assurer que le chirurgien placera exactement aux bons endroits, sur la ou sur les deux mâchoires du patient, les plaques d'ostéosynthèse préparées sur mesure par avance.

La présente invention a pour but d'apporter une solution à ce problème en proposant un ensemble sur mesure d'au moins deux plaques d'ostéosynthèse, elles-mêmes préparées sur mesure.L'invention est définie dans les revendications annexées.

Une telle structure d'ensemble se caractérise par le fait que les plaques d'ostéosynthèse sont reliées entre elles par une tige. Dans les exemples non réclamés, laquelle tige comporte en outre deux proéminences qui émergent de ladite tige en direction de l'orifice nasal du patient en des positions qui correspondent aux deux extrémités de la base de l'orifice nasal du patient. Dans ces exemples non réclamés, les coins inférieurs de l'orifice nasal du patient ne pouvant varier en position, le fait pour le chirurgien de les utiliser comme références naturelles pour loger les proéminences de la structure d'ensemble selon l'invention et, à cette suite, pour placer ladite structure d'ensemble soit sur la mâchoire inférieure, soit sur la mâchoire supérieure du patient concernée par l'opération, garantit que toutes les plaques d'ostéosynthèse de ladite structure occuperont sans aucune exception les emplacements qu'il a par avance été prévu qu'elles tiennent.

Avantageusement, les deux proéminences ont chacune la forme d'un téton, d'un doigt, d'un ergot ou d'un éperon.

Les deux repères en saillie ainsi prévus par construction sur la tige de la structure d'ensemble selon l'invention étant des proéminences qui viennent se nicher dans les coins inférieurs de l'orifice nasal du patient, la pose par le chirurgien de ladite structure d'ensemble, avec la plus grande précision qui soit, en est facilité.

Toujours avantageusement, la tige de la structure d'ensemble selon l'invention respecte elle-aussi parfaitement l'anatomie du patient.

Dans une autre forme préférée de réalisation, la structure d'ensemble selon l'invention se caractérise par le fait que, à proximité de chacune de ses plaques, la tige de ladite structure présente des zones de moindre résistance destinées à faciliter la coupe par le chirurgien de toutes les parties de liaison entre les différentes plaques d'ostéosynthèse de ladite structure.

Après son placement en position idéale, par le chirurgien, de la structure d'ensemble selon l'invention, puis fixation de toutes les plaques d'ostéosynthèse par vissage dans l'os de la mâchoire supérieure ou de la mâchoire inférieure du patient, il est à l'évidence souhaitable de faire immédiatement disparaître toutes les parties de liaison entre lesdites plaques, y compris la partie qui présente les proéminences ayant permis la mise précise en position idéale de la structure d'ensemble sur la mâchoire du patient. Les zones de moindre résistance prévues à proximité immédiate des plaques ne feront que simplifier la tâche du chirurgien tout au long de cette opération d'élimination des parties de la structure d'ensemble selon l'invention devenues inutiles.

La structure d'ensemble selon l'invention est avantageusement fabriquée d'une seule pièce et, de préférence, elle est réalisée en titane. Un exemple non réclamé, a également pour objet un procédé de mise en place d'au moins deux plaques d'ostéosynthèse sur le maxillaire et/ou sur la mandibule d'un patient, lors d'une opération d'occlusion dentaire, ledit procédé étant caractérisé en ce que, en préliminaire de ladite opération, l'on simule par avance les résultats de l'opération sur le patient, l'on détermine la position et la forme des plaques d'ostéosynthèse en fonction de la future anatomie du patient, l'on réalise sur mesure un ensemble d'au moins deux plaques d'ostéosynthèse, par exemple de types droites en I et/ou en L et/ou en croix et/ou en forme de patte-d'oie, elles-mêmes réalisées sur mesure en fonction de l'opération à réaliser et de la future anatomie du patient, ledit ensemble comprenant une tige qui relie entre elles les plaques d'ostéosynthèse et qui comporte en outre deux proéminences correspondant aux coins inférieurs de l'orifice nasal du patient, et en ce que, après l'opération, le chirurgien place idéalement ledit ensemble sur mesure en disposant ses deux proéminences dans les coins inférieurs de l'orifice nasal du patient, puis il fixe toutes les plaques d'ostéosynthèse à l'aide de vis pénétrant les éléments osseux de la mâchoire supérieure ou de la mâchoire inférieure concernée, puis il coupe à proximité de chacune des plaques d'ostéosynthèse dudit ensemble toutes les parties de liaison formées par la tige entre lesdites plaques.

L'invention sera mieux comprise à la lecture de la description qui va suivre, et en référence aux dessins annexés qui illustrent de manière non limitative un mode de réalisation de ladite invention, et sur lesquels :
- les Figures 1 et 2 représentent, vu de face et respectivement vu de côté, un exemple de réalisation de la structure d'ensemble sur mesure de plaques d'ostéosynthèse selon l'invention, ladite structure d'ensemble comportant quatre plaques en L dans un tel exemple.
- la Figure 3 illustre en vue de face le placement en position idéale sur la mâchoire supérieure d'un patient de l'ensemble sur mesure des Figures 1 et 2.
- la Figure 4 illustre en vue de profil le même placement en position idéale de l'ensemble sur mesure des Figures 1 et 2 sur la mâchoire supérieure du patient.

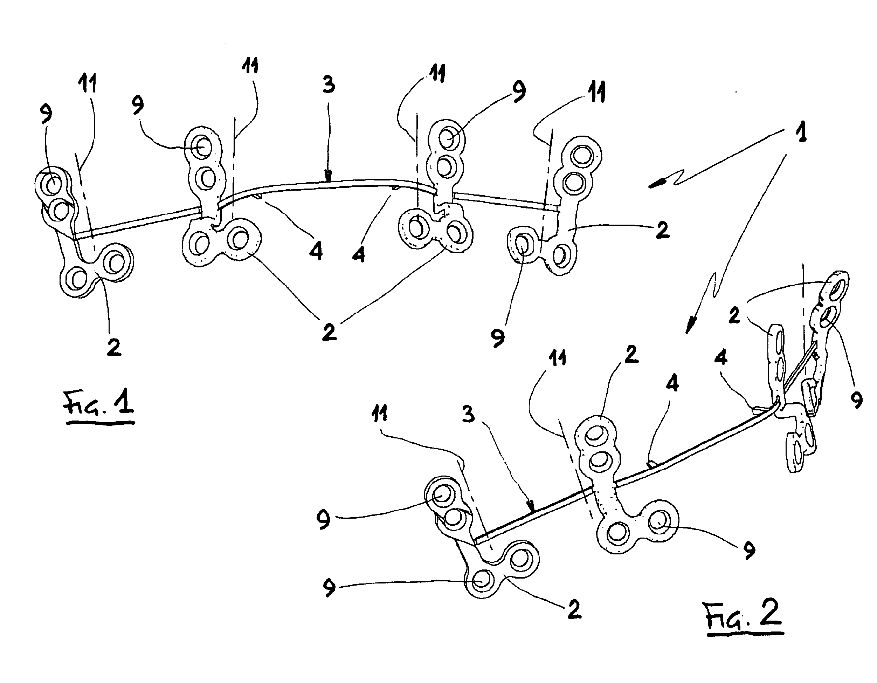

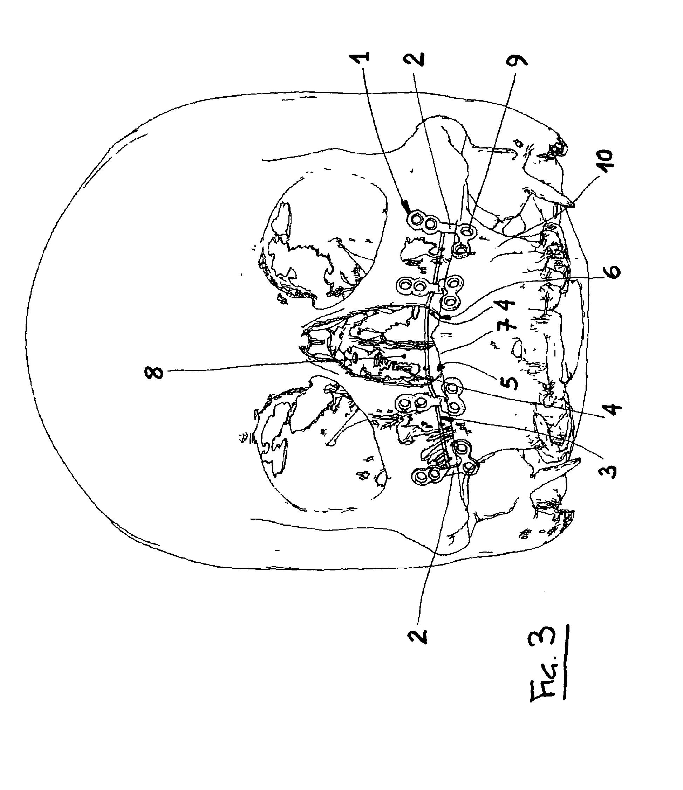

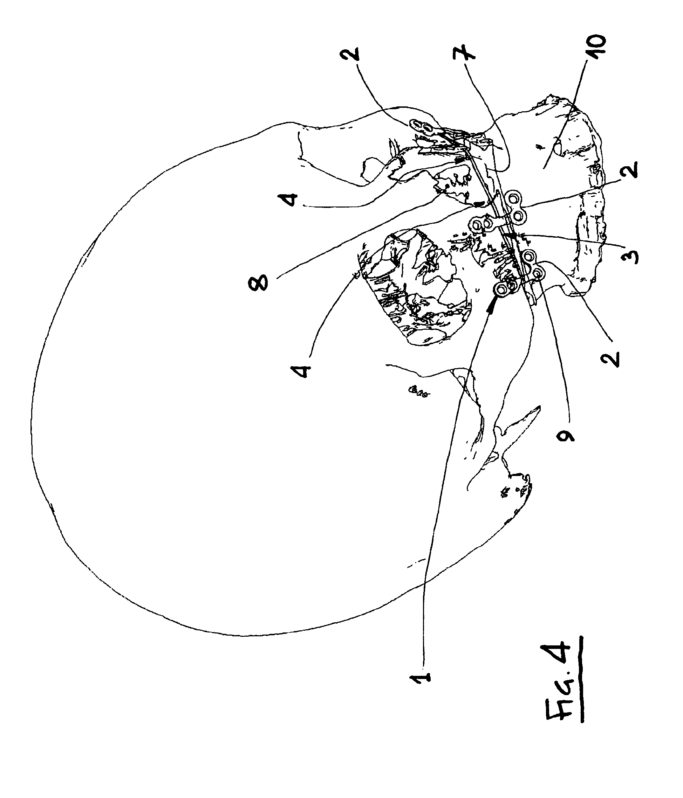

Dans l'exemple choisi, représenté sur les Figures 1 à 4, l'opération d'occlusion dentaire réalisée porte exclusivement sur la mâchoire supérieure, encore appelée maxillaire.

En variante, il est toutefois bien clair que l'invention s'applique également à toute réparation de la mâchoire inférieure, encore appelée mandibule, que cette réparation soit réalisée seule ou concomitamment avec celle de la mâchoire supérieure.

Dans cette variante, seules seront modifiées la forme de la tige de liaison ainsi que les formes des plaques d'ostéosynthèse qui, pour la réparation du maxillaire, sont le plus souvent du type en L et/ou en croix et/ou en forme de patte-d'oie.

En effet, pour la réparation de la mandibule, il leur est généralement préféré des plaques d'ostéosynthèse en forme de I, c'est-à-dire des plaques droites.

Connaissant la future anatomie du patient à partir des données d'un scanner ou d'une IRM, on réalise conformément à l'invention, en préliminaire de l'opération convenue, un ensemble 1 d'au moins deux plaques d'ostéosynthèse, quatre en L dans l'exemple représenté sur les Figures 1 à 4, qui est formé :
- des plaques d'ostéosynthèse 2 à proprement parler, lesquelles, en fonction précisément de la future anatomie du patient, et donc des positions qu'elles devront occuper sur sa mâchoire à réparer, sont mises chacune à la forme appropriée, c'est-à-dire sur mesure, avec ici et là les courbures qui leur garantiront d'épouser parfaitement la future anatomie désirée,
- une tige 3 qui relie entre elles toutes les plaques 2, ladite tige comportant en outre deux proéminences 4 correspondant très exactement aux deux extrémités, respectivement 5 et 6, de la base 7 de l'orifice nasal 8 du patient.

Chaque plaque d'ostéosynthèse 2 est, de façon connue, percée de plusieurs trous traversants 9, ici quatre trous pour chaque plaque en forme de L, lesquels trous sont destinés à recevoir les vis qui, après la mise en place parfaite de l'ensemble 1 (encore appelé structure d'ensemble) sur le maxillaire 10 du patient, assureront la fixation dudit ensemble sur ledit maxillaire en étant vissées, par le chirurgien dans les parties osseuses qui, disjointes après l'opération, doivent être réunies en étant placées les unes contre les autres.

Les deux proéminences 4 sont par exemple avantageusement en forme de tétons ou de doigts, ou d'ergots, ou d'éperons, lesquels émergent de la tige 3 en direction de l'orifice nasal 8 du patient. Toujours par construction, les positions sur la tige et les formes de ces proéminences 4 ont été déterminées à partir des données connues des scanners ou IRM.

De préférence, la tige 3 respecte elle-aussi parfaitement l'anatomie de l'os maxillaire ou, selon, celle de l'os mandibulaire du patient ; ses parties courbes et rectilignes sont donc connues à partir des données précitées et elles sont ainsi reproduites à l'identique sur la tige lors de la construction de la structure d'ensemble.

Après avoir effectué l'opération désirée, le chirurgien place les unes contre les autres les parties osseuses disjointes, puis il met en place la structure 1 en disposant les deux proéminences 4 dans les coins inférieurs 5 et 6 de l'orifice nasal 8 du patient, c'est-à-dire en les logeant aux deux extrémités de la base 7 dudit orifice nasal.

La structure 1 occupe idéalement sa place, déterminée à l'avance, par rapport au maxillaire du patient et le chirurgien n'a plus alors qu'à procéder à la fixation de ladite structure sur le maxillaire à l'aide des vis traversant les trous 9 des plaques d'ostéosynthèse 2 en L.

Les parties osseuses disjointes étant ainsi réunies fixement, et devant l'être jusqu'à la cicatrisation de l'os, le chirurgien n'a plus qu'à éliminer de la structure 1 toutes les parties de celle-ci devenues inutiles, c'est-à-dire les parties de liaison entre les différentes plaques d'ostéosynthèse 2, seules lesdites plaques demeurant donc dans la bouche du patient en fin d'opération.

Pour cela, le chirurgien coupe la tige 3 à proximité immédiate de chacune des plaques 2, y compris la partie de la tige qui comprend les deux proéminences 4.

Afin de faciliter ce travail de coupe de la tige en vue de son élimination, on prévoit par construction que ladite tige présente au voisinage de chacune des différentes plaques 2 des zones de moindre résistance illustrées par les traits mixtes 11, ces derniers, pour une meilleure clarté des dessins, n'ayant été schématisés qu'en quelques occasions.

L'ensemble sur mesure 1 selon l'invention - plaques d'ostéosynthèse 2 et tige 3 - est avantageusement fabriqué en titane, matériau biocompatible d'usage maintenant courant en chirurgie, et ledit ensemble est de préférence réalisé d'une seule pièce.

Comme il va de soi, l'invention ne se limite pas aux seules spécifications techniques données ci-dessus à titre d'exemples ; elle embrasse, au contraire, toutes les variantes possibles de réalisation, en particulier celle de la construction d'une autre structure 1 adaptée spécifiquement à la réparation d'une mandibule, autre structure pour laquelle, d'une part, la tige 3, au lieu d'être sensiblement rectiligne, aura davantage la forme d'un oméga et, d'autre part, des plaques 2 en forme de I viendront selon la tradition se substituer aux plaques choisies dans le présent exemple en forme de L, mais qui pourraient aussi bien être en croix ou en forme de patte-d'oie.

Les clauses suivantes ne sont pas réclamés, mais décrivent possibles exemples non réclamés:
1) Ensemble sur mesure (1) d'au moins deux plaques d'ostéosynthèse (2), par exemple de types droites en I et/ou en L et/ou en croix et/ou en forme de patte-d'oie, elles-mêmes préparées sur mesure en fonction de l'opération à réaliser et de la future anatomie du patient, caractérisé en ce que les plaques d'ostéosynthèse (2) sont reliées entre elles par une tige (3), laquelle tige comporte en outre deux proéminences (4) qui émergent de ladite tige (3) en direction de l'orifice nasal (8) du patient en des positions qui correspondent aux deux extrémités (5, 6) de la base (7) de l'orifice nasal (8) du patient.
2) Ensemble sur mesure (1) d'au moins deux plaques d'ostéosynthèse (2) selon la clause 1, caractérisé en ce que les deux proéminences (4) ont la forme de tétons, ou de doigts, ou d'ergots, ou d'éperons.
3) Ensemble sur mesure (1) d'au moins deux plaques d'ostéosynthèse (2) selon l'une quelconque des clauses 1 et 2, caractérisé en ce que la tige (3) dudit ensemble respecte elle-aussi parfaitement l'anatomie du patient.
4) Ensemble sur mesure (1) d'au moins deux plaques d'ostéosynthèse (2) selon l'une quelconque des clauses 1 à 3, caractérisé en ce que, à proximité de chacune de ses plaques (2), la tige (3) dudit ensemble présente des zones de moindre résistance (11) destinées à faciliter la coupe par le chirurgien de toutes les parties de liaison entre les différentes plaques d'ostéosynthèse dudit ensemble.
5) Ensemble sur mesure (1) d'au moins deux plaques d'ostéosynthèse (2) selon l'une quelconque des clauses 1 à 4, caractérisé en ce qu'il est fabriqué d'une seule pièce.
6) Ensemble sur mesure (1) d'au moins deux plaques d'ostéosynthèse (2) selon l'une quelconque des clauses 1 à 5, caractérisé en ce qu'il est réalisé en titane.
7) Procédé de mise en place d'au moins deux plaques d'ostéosynthèse (2) sur le maxillaire (10) et/ou sur la mandibule d'un patient, lors d'une opération d'occlusion dentaire, caractérisé en ce que, en préliminaire de ladite opération, l'on simule par avance les résultats de l'opération sur le patient, l'on détermine la position et la forme des plaques d'ostéosynthèse (2) en fonction de la future anatomie du patient, l'on réalise sur mesure un ensemble (1) d'au moins deux plaques d'ostéosynthèse, par exemple de types droites en I et/ou en L et/ou en croix et/ou en forme de patte-d'oie, elles-mêmes réalisées sur mesure en fonction de l'opération à réaliser et de la future anatomie du patient, ledit ensemble (1) comprenant une tige (3) qui relie entre elles les plaques d'ostéosynthèse (2) et qui comporte en outre deux proéminences (4) correspondant aux coins inférieurs (5, 6) de l'orifice nasal (8) du patient, et en ce que, après l'opération, le chirurgien place idéalement ledit ensemble sur mesure (1) en disposant ses deux proéminences (4) dans les coins inférieurs (5, 6) de l'orifice nasal (8) du patient, puis il fixe toutes les plaques d'ostéosynthèse (2) à l'aide de vis pénétrant les éléments osseux de la mâchoire supérieure ou de la mâchoire inférieure concernée, puis il coupe à proximité de chacune des plaques d'ostéosynthèse (2) dudit ensemble toutes les parties de liaison formées par la tige (3) entre lesdites plaques (2).
8) Procédé selon la clause 7, caractérisé en ce que le chirurgien coupe toutes les parties de liaison entre les plaques d'ostéosynthèse (2) formées par la tige (3) de l'ensemble sur mesure (1), en utilisant à cette fin des zones de moindre résistance (11) prévues à proximité de chacune desdites plaques.

## Revendications

1. Ensemble (1) pour l'ostéosynthèse d'une mâchoire, comprenant :
au moins deux plaques d'ostéosynthèse (2), chaque plaque d'ostéosynthèse comprenant une première portion pour la fixation à une première partie d'os de la mâchoire et une deuxième portion pour la fixation à une deuxième partie d'os de la mâchoire, lesdites plaques d'ostéosynthèse étant destinées à une utilisation dans l'orientation de la deuxième partie d'os en relation avec la première partie d'os,
l'ensemble étant **caractérisé en ce que** :
chaque plaque d'ostéosynthèse (2) a une courbure préconfigurée en relation avec une position que la plaque occupera lorsqu'elle sera fixée aux première et deuxième parties d'os de la mâchoire, la courbure étant ajustée sur la base d'une future anatomie souhaitée de la mâchoire,
et **en ce que** l'ensemble comprend :
une partie de raccordement (3) pour raccorder conjointement lesdites plaques d'ostéosynthèse, la partie de raccordement ayant une forme préconfigurée en relation avec au moins deux positions que lesdites plaques d'ostéosynthèse occuperont lorsqu'elles seront fixées aux première et deuxième parties d'os de la mâchoire, la forme étant ajustée sur la base d'une future anatomie souhaitée de la mâchoire.

2. Procédé de production d'un ensemble (1) pour l'ostéosynthèse, comprenant :
la consultation de données indicatives d'une future anatomie souhaitée d'un patient,
le procédé étant **caractérisé par** :
la fabrication d'au moins deux plaques d'ostéosynthèse (2), chaque plaque d'ostéosynthèse comprenant une première portion pour la fixation à une première partie d'os du patient et une deuxième portion pour la fixation à une deuxième partie d'os du patient, lesdites plaques d'ostéosynthèse étant destinées à une utilisation dans l'orientation de la deuxième partie d'os en relation avec la première partie d'os,
ladite fabrication comprenant :
la définition d'une courbure préconfigurée en relation avec une position que la plaque occupera lorsqu'elle sera fixée aux première et deuxième parties d'os du patient, la courbure étant ajustée sur la base des données consultées, et
la définition d'une partie de raccordement (3) pour raccorder conjointement lesdites plaques d'ostéosynthèse, la partie de raccordement ayant une forme préconfigurée en relation avec au moins deux positions que lesdites plaques d'ostéosynthèse occuperont lorsqu'elles seront fixées aux première et deuxième parties d'os du patient, la forme étant ajustée sur la base des données consultées,
dans lequel l'ensemble (1) est fabriqué selon lesdites définitions.

3. Ensemble (1) selon la revendication 1 ou procédé selon la revendication 2, dans lequel les première et deuxième parties d'os correspondent respectivement aux parties d'os supérieure et inférieure d'une mâchoire.

4. Ensemble (1) selon l'une quelconque des revendications 1 et 3, ou procédé selon l'une quelconque des revendications 2 et 3, dans lequel les première et deuxième parties d'os sont des parties d'un maxillaire ou d'une mandibule.

5. Ensemble (1) selon l'une quelconque des revendications 1 et 3 ou 4, ou procédé selon l'une quelconque des revendications 2 à 4, dans lequel chacune des première et deuxième portions de chaque plaque d'ostéosynthèse comprend au moins une ouverture pour fixer la portion à une partie d'os respective.

6. Ensemble (1) selon l'une quelconque des revendications 1 et 3 à 5, ou procédé selon l'une quelconque des revendications 2 à 5, dans lequel la partie de raccordement comprend une portion de référence pour l'orientation de l'ensemble par rapport à au moins l'une des première et deuxième parties d'os.

7. Ensemble (1) selon l'une quelconque des revendications 1 et 3 à 6, ou procédé selon l'une quelconque des revendications 2 à 6, dans lequel la forme de la partie de raccordement comprend une configuration d'une ou plusieurs parties courbées et rectilignes de la partie de raccordement.

8. Ensemble (1) selon l'une quelconque des revendications 1 et 3 à 7, ou procédé selon l'une quelconque des revendications 2 à 7, dans lequel la partie de raccordement comprend des zones de résistance réduite pour son utilisation dans le retrait de la partie de raccordement.

9. Procédé selon l'une quelconque des revendications 2 à 8, dans lequel les données indicatives d'une future anatomie souhaitée d'un patient sont générées sur la base de données provenant d'un scanner ou d'une imagerie à résonance magnétique (IRM).

10. Procédé selon l'une quelconque des revendications 2 à 9, dans lequel les données indicatives d'une future anatomie souhaitée d'un patient sont générées sur la base de données provenant d'une opération simulée réalisée sur le patient.

11. Procédé selon l'une quelconque des revendications 2 à 10, comprenant :
la détermination d'une ou plusieurs positions de portions de référence sur la base de données provenant d'un scanner ou d'une imagerie à résonance magnétique (IRM).

12. Ensemble (1) pour l'ostéosynthèse comprenant :
au moins deux plaques d'ostéosynthèse (2),
l'ensemble étant **caractérisé en ce que** :
chaque plaque d'ostéosynthèse est ajustée sur la base d'une future anatomie souhaitée,
et **en ce que** l'ensemble comprend :
une partie de raccordement pour raccorder conjointement lesdites plaques d'ostéosynthèse, la partie de raccordement ayant une forme préconfigurée en relation avec au moins deux positions que lesdites plaques d'ostéosynthèse occuperont lorsqu'elles seront fixées à une ou plusieurs parties d'os, la forme étant ajustée sur la base d'une future anatomie souhaitée,
la partie de raccordement comprenant des zones de résistance réduite pour son utilisation dans le retrait de la partie de raccordement.
